# EUROPEAN PATENT APPLICATION

(11) **EP 4 767 984 A1**
(43) Date of publication of application: **01.07.2026**
(21) Application number: 24855843.9
(22) Date of filing: 21.08.2024
(51) Int. Cl.: A61B 34/30, A61B 34/20, A61B 34/32

(54) **SURGICAL ROBOT FOR ORTHOPEDIC SURGERY**

(30) Priority: 24.08.2023 CN 202311073132
(71) Applicant: CHUNFENGHUAYU (SUZHOU) INTELLIGENT MEDICAL TECHNOLOGY CO., LTD., Taicang Suzhou, Jiangsu 215413 (CN)
(72) Inventor: YOUNG, Victor, Suzhou, Jiangsu 215413 (CN); CHEN, Chaoliang, Suzhou, Jiangsu 215413 (CN); SHI, Weisong, Suzhou, Jiangsu 215413 (CN)
(74) Representative: Maiwald GmbH
(86) International application number: PCT/CN2024/113667
(87) International publication number: WO 2025/040121

(57) **Abstract**

The present disclosure relates to a surgical robot for orthopedic surgery, comprising a robotic arm device, a positioning and navigation device, and a control device, wherein the robotic arm device comprises at least two robotic arms, and an end effector is arranged at the end of each robotic arm close to an operating zone. The positioning and navigation device comprises an electromagnetic navigation device. The electromagnetic navigation device comprises: a tracking apparatus for electromagnetic tracking, comprising a magnetic field generator used for covering the at least two end effectors and at least two first electromagnetic tracking elements used for respectively tracking the orientations of the at least two end effectors during the surgery; and a reference frame, wherein a second electromagnetic tracking element and a developing body capable of displaying a coordinate position are arranged on the reference frame. The positioning and navigation device further comprises an imaging device used for positioning and navigating the at least two end effectors. The imaging device comprises an infrared coherent imaging navigation device used for positioning the operating zone and the at least two end effectors.

## Description

For all purposes, the present application claims priority to Chinese Patent Application No. 202311073132.2 filed on August 24, 2023, the entire content of which is incorporated herein by reference as part of the present application.

### TECHNICAL FIELD

The present disclosure relates to a surgical robot for orthopedic surgery, including a robotic arm device, a positioning-and-navigation device, and a control device.

### BACKGROUND

Surgical robots are used to assist surgery, to customize three-dimensional preoperative plans, provide clearer and more precise visualization of surgical sites, reduce tremors to improve surgical accuracy, minimize damage to healthy bones and tissues, reduce blood loss, protect nerves, shorten hospital stays, and accelerate recovery. They can also guide remote surgery and reduce intraoperative radiation imaging (such as X-rays) to reduce radiation exposure.

Robot-assisted orthopedic surgery is an important application of orthopedic surgical robots. In the past, surgeons placed screws in the spine bone to perform complex spinal surgeries manually or with the assistance of numerous intraoperative X-ray images, resulting in a risk of radiation exposure for both patients and surgeons. Furthermore, spinal surgery requires high precision in screw placement, and the consequences of placing screws in incorrect or suboptimal positions are severe.

Orthopedic surgical robot can provide a guidance system based on computerized preoperative planning, which significantly improves accuracy and reduces the risk of screw misplacement. However, existing orthopedic surgical robots generally have only a single arm or dual arms, which are used for intraoperative navigation and/or Kirschner wire placement. In conventional orthopedic robot spinal pedicle screw placement surgery procedures, a guide wire is inserted according to intraoperative planning, followed by the placement of a hollow pedicle screw through the guide wire; ultimately, the procedure still relies on manual drilling and screw placement by the surgeon, resulting in low screw placement efficiency. Existing orthopedic surgical robot is generally equipped with an infrared navigation system, which is affected by obstruction by intraoperative items and movement of the patient or patient support device, leading to navigation deviations, surgical interruptions, and other issues.

### SUMMARY

The technical problem to be solved by the present disclosure is to provide an improved surgical robot for orthopedic surgery, which greatly enhances a degree of automation in the orthopedic surgery, significantly improves operational efficiency and surgical accuracy of an existing orthopedic procedures and at the same time substantially reduces the workload of the orthopedic surgeon.

This technical problem is solved by a surgical robot for orthopedic surgery, which includes: a robotic arm device, a positioning-and-navigation device, and a control device that is communicatively coupled with the robotic arm device and the positioning-and-navigation device, respectively. According to the present disclosure, the robotic arm device is provided with at least two robotic arms, an end effector is mounted at an end of each of the at least two robotic arms proximate to an surgical site, the positioning-and-navigation device includes a tracking device for electromagnetic tracking, the tracking device including: a magnetic field generator configured to cover at least two end effectors, and at least two first electromagnetic tracking elements for respectively tracking orientations of the at least two end effectors during a surgery; and a reference frame, where a second electromagnetic tracking element and a marker that is capable of displaying a coordinate position are mounted on the reference frame. The positioning-and-navigation device further includes an imaging device for positioning and navigating the at least two end effectors, and the imaging device is provided with an infrared coherent imaging navigation device for positioning the surgical site and the at least two end effectors. Thus, both drilling and screw placement by the surgical robot during the orthopedic surgery are not affected by movements of a patient and a surgical table.

To perform various types of orthopedic surgeries with high precision, including minimally invasive and open surgeries, particularly spinal screw-placement surgery, the present disclosure establishes a dual-navigation system including infrared coherent imaging navigation and electromagnetic navigation. Electromagnetic navigation is applicable to both the minimally invasive and open surgeries. Although the infrared coherent imaging navigation is more suitable for an open surgery because of its imaging depth limitation, its high-resolution imaging capability enables ultra-high-precision navigation. For the infrared coherent imaging navigation device, optical design is first employed to achieve a coherent distance of more than 1 meter, thereby enabling long-distance real-time coherent imaging of the surgical site and surgical tools (or surgical instruments). Tracking and navigation are achieved after the infrared coherent imaging navigation device is coordinate-registered with the robotic arm. For the electromagnetic navigation system, firstly, an electromagnetic navigation reference frame and supporting surgical tools are designed, and position data of the surgical site and surgical tools are acquired by electromagnetic detection elements to implement tracking, and navigation is achieved after the registration with CT imaging coordinates and robotic arm system coordinates. Real-time navigation registration can be realized by developing the dual-navigation system through algorithms, thereby suppressing drift caused by patient respiration.

The infrared coherent imaging navigation does not require intraoperative CT imaging, thereby reducing X-ray radiation exposure for both medical staff and patients, while also enabling precise robotic positioning and anatomical visualization in the surgical site to assist surgeons or the surgical robot in performing the surgery during a surgery procedure.

In a further extended embodiment, an advantageous manner to achieve improved positioning, navigation and monitoring effects is that the imaging device is arranged at an end of one of the at least two robotic arms that is proximate to the surgical site, and the imaging device is communicatively connected to the control device, so as to maintain the imaging device in a working position and orientation throughout a surgical procedure. Thus, the control device of the surgical robot can adjust the position and the orientation of the imaging device in accordance with the movement of the surgical site, so as to achieve optimal monitoring, positioning and navigation effects.

According to an extended design of the present disclosure, more precise electromagnetic positioning can be achieved by arranging the first electromagnetic tracking elements in or on the at least two end effectors respectively, because the orientation of an end effector relative to the corresponding robotic arm may be inconsistent for various reasons when the end effector is mounted on the robotic arm.

Advantageously, to facilitate surgical monitoring and learning by medical staff during a surgery, avoid conflicts with other surgical instruments and apparatus, and eliminate the need for sterile draping, the imaging device further includes a remote microscopic device for imaging and tracking an object in the surgical site. The remote microscopic device is arranged at a position more than 1 meter above a patient support device. By arranging the remote microscopic device above the patient support device, it can be ensured that the remote microscopic device does not obstruct other intraoperative procedures while maintaining continuous imaging.

In a further extended embodiment, the control device is configured to perform the tracking by an identifier for the surgical site. A more convenient and easily implementable manner of tracking is to provide an identifier that is used for identifying the position of the surgical site, thereby tracking can be realized by recognizing the identifier used to identify the position of the surgical site.

Further, to facilitate controlling orientations of the at least two robotic arms, the control device is configured to control the orientations of the at least two robotic arms based on a distance of the identifier and an orientation of the identifier.

To further facilitate monitoring and learning by medical staff during the surgery, the positioning-and-navigation device further includes a monitoring device with a plurality of monitors. One of the plurality of monitors is a first monitor for displaying data from the infrared coherent imaging navigation device and the electromagnetic navigation device, and another one of the plurality of monitors is a second monitor for displaying data from the remote microscopic device.

In a further extended embodiment, the remote microscopic device is configured for stereoscopic visualization of the surgical site to perceive the surgical site in depth preoperatively, for example, the natural depth perception of the spine to be operated on that is required prior to complex spinal surgery. This is similar to glasses-free 3D, where a glasses-free 3D spinal image is overlaid on a skin surface, enabling the surgeon to visualize the position of the bone beneath the skin preoperatively.

Advantageously for the automation of robotic surgery, the at least two robotic arms include a first robotic arm for mounting a first end effector, a second robotic arm for mounting a second end effector, and a third robotic arm for mounting the imaging device.

The remote microscopic device and the infrared coherent imaging navigation device are arranged on the third robotic arm, so as to enable a visual field of the remote microscopic device and a visual field of the infrared coherent imaging navigation device to coincide, particularly the visual fields of their probes coincide. Thus, this enables the remote microscopic device and the infrared coherent imaging navigation device to be simultaneously aligned with the surgical site where the orthopedic surgery is to be performed.

In a further extended embodiment, the first end effector is configured as a first implant-inserter for implanting a first implant into a patient, and the second end effector is selectively configured as either a second implant-inserter for implanting a second implant into a patient or a drilling tool. In order to reduce the load on the control device in analyzing and processing data from each robotic arm while ensuring a high level of automation and good performance of the surgical robot, the number of robotic arms should be minimized as much as possible. It is particularly advantageous for this purpose that the second end effector is selectively configured as either the second implant-inserter for implanting the second implant into the patient or the drilling tool. Therefore, this eliminates the need to provide one robotic arm for each surgical instrument. This selective configuration is achieved by replacing the drilling tool used for a drilling operation with another surgical instrument such as an implant-inserter, after the surgical robot has performed the drilling operation through the second robotic arm, so that the same robotic arm, i.e., the second robotic arm, can perform several surgical operations.

In a further extended embodiment, in the case where the second end effector is configured as a second implant-inserter, the first implant-inserter cooperates with the second implant-inserter during the surgical procedure, so as to enable torques exerted by the first implant-inserter and the second implant-inserter on the surgical site to be balanced. Therefore, this improves the stability and success rate of implant placement in the orthopedic surgery, and is particularly applicable in scenarios where the placement of left-handed and right-handed screws is required, for example, where the bone is very hard and using only right-handed screw may cause secondary injuries such as fractures.

In a further extended embodiment, the first implant is configured as one of a left-handed pedicle screw and a right-handed pedicle screw, and the second implant is configured as the other one of the left-handed pedicle screw and the right-handed pedicle screw. Simultaneous-screw-placement and cancellation of left-and-right torques, by using the dual robotic arms, further improve the stability and the success rate of the orthopedic surgery, particularly the stability and the success rate of spinal screw placement.

The control device of the surgical robot according to the present disclosure includes: a surgical planning module configured for planning a surgery; a data-analysis-and-processing module configured for analyzing and processing data to control the robotic arm device; and a robotic arm control module configured for controlling movement of the robotic arm device. In an advantageous embodiment of the present disclosure, the surgical planning module is configured to select and save an entry point and a trajectory for a planned orthopedic surgical operation during a planning phase.

In a further extended design, in order to determine the motion trajectory of the corresponding robotic arm, it is advantageous that the data-analysis-and-processing module is configured to share, in a form of coordinate transformation, a position of the entry point and an orientation of the trajectory for the orthopedic surgical operation that are selected and saved with the robotic arm control module.

More advantageously for the control of the surgical robot, the robotic arm control module is further configured to define motion orientations of the at least two robotic arms by angles and Cartesian coordinates.

In order not to interfere with the orthopedic surgery robot and/or surgical participants, the magnetic field generator is arranged on the patient support device. In particular, the magnetic field generator may be integrated into any position on the surgical table, provided that it does not interfere with the movement of the orthopedic surgical robot or some typical preoperative or intraoperative workflows in an operating room.

The examples will be described in detail below in conjunction with the accompanying drawings, and further details and advantages of the examples described in detail herein will become clear.

### BRIEF DESCRIPTION OF DRAWINGS

Fig. 1 is a schematic diagram of a non-limiting embodiment of a robot for orthopedic surgery according to the present disclosure;
Fig. 2 is a block diagram illustrating the working principle of a robot for orthopedic surgery according to the present disclosure;
Figs. 3a-3b show non-limiting embodiments of a first implant and a second implant according to the present disclosure, respectively.

Referring to the accompanying drawings, where same reference numerals refer to same components throughout the several views, and the present disclosure relates to a surgical robot for orthopedic surgery.

### DETAILED DESCRIPTION

It should also be understood that the specific apparatuses, devices and systems illustrated in the accompanying drawings and described in the following specification are merely exemplary illustrations of the present disclosure. Accordingly, the specific dimensions and other physical characteristics associated with the examples disclosed herein shall not be construed as restrictive.

The technical term "orthopedic surgery" as used in the present disclosure includes, but is not limited to, laminectomy, discectomy, vertebroplasty, lumbar fusion and the like. The ordinal terms "first", "second" and "third" are used merely to mean different parts and do not imply any limitation as to order.

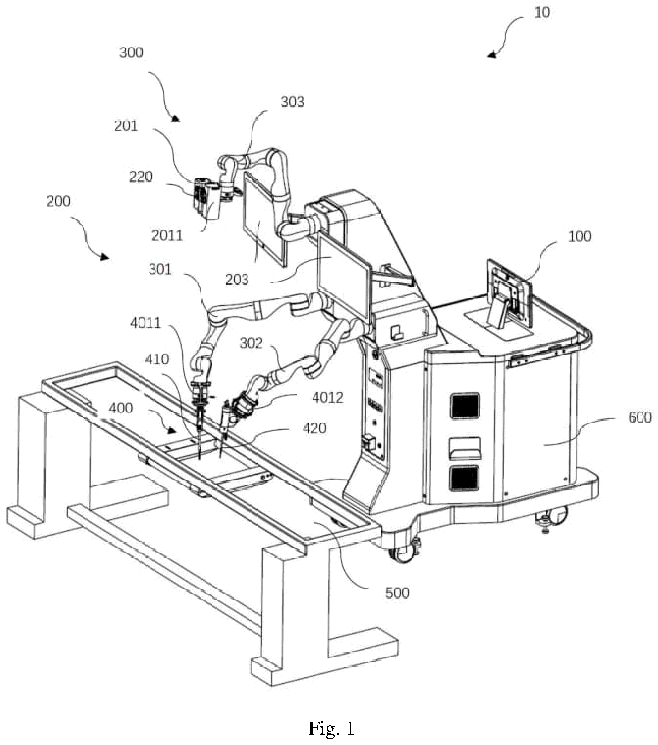

Fig. 1 illustrates a non-limiting embodiment of a surgical robot 10 for orthopedic surgery according to the present disclosure. The surgical robot 10 provided by the present disclosure mainly includes a control device 100, a positioning-and-navigation device 200 and a robotic arm device 300, and generally additionally includes a tool set for the orthopedic surgery, a patient support device 500 for a patient to be operated on, and a cart device 600 for at least fixing the robotic arm device 300.

In one non-limiting embodiment of the present disclosure, the robotic arm device 300 is provided with at least two robotic arms, preferably three robotic arms, namely a first robotic arm 301, a second robotic arm 302 and a third robotic arm 303. An end effector 400 for performing a surgery, in particular the orthopedic surgery, may be detachably mounted on an end of each of the first robotic arm 301 and the second robotic arm 302 that is proximate to a surgical site, and an imaging device 201 is fixedly mounted on a free end of the third robotic arm 303. Additionally, the robotic arm device 300 may also be used as a mechanical driver for the two end effectors 400. To obviate the need for medical staff to replace the end effector 400 during a surgical procedure and thereby improving the operational efficiency of the surgical robot 10, the three robotic arms may be configured respectively as the first robotic arm 301 for mounting a first end effector 410, the second robotic arm 302 for mounting a second end effector 420, and the third robotic arm 303 for mounting the imaging device 201, where each robotic arm is preferably implemented as a multi-axis robotic arm as shown in Fig. 1.

Of course, depending on the complexity of surgical conditions, the surgical robot may also be modified to be provided with a total of more than three robotic arms operating in coordination, thereby facilitating more efficient surgical execution. Conversely, in order to reduce the load of data-analysis-and-processing by the control device 100, the surgical robot may alternatively be modified to include only a single robotic arm, with a port for connecting different end effectors that is formed at the free end of the robotic arm. Unlike the surgical robot with three robotic arms, this robot with only one such robotic arm requires medical staff to manually replace the end effector during the surgical procedure. Consequently, in the case of providing a single robotic arm, the surgical robot achieves a lower degree of automation in performing the surgical procedure than in the case of providing three robotic arms.

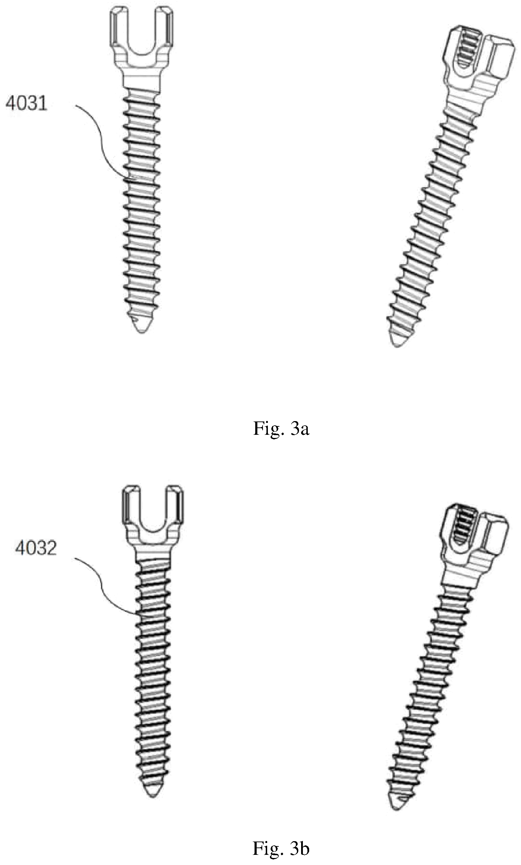

The first end effector 410 and the second end effector 420 mounted on the first robotic arm 301 and the second robotic arm 302 respectively may be implemented either as surgical tools for performing surgical operations (such as an osteotome, a screw driver, a drill bit, a retractor, a bone forceps, a bone mallet, or other types of surgical tools), or as an implant-inserter for implanting an implant (such as a screw driver). In a non-limiting embodiment of the present disclosure, the first end effector 410 may be configured as a first implant-inserter for implanting a first implant as shown in Fig. 3a into a human body, and the second end effector 420 may be optionally configured as a surgical tool in the form of a drilling tool for drilling a hole or as a second implant-inserter for implanting a second implant as shown in Fig. 3b into a human body. The implant-inserter and the drilling tool will be further described below.

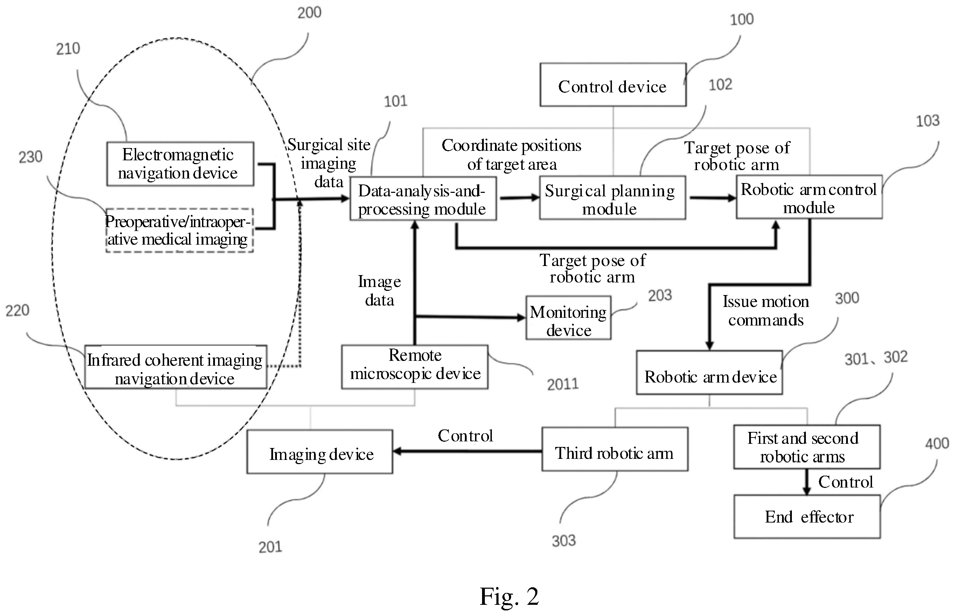

The first robotic arm 301 and the second robotic arm 302 position and orient the end effectors 400 of the robot according to the commands issued by the surgical planning module 102 and the tracking device as shown in Fig. 2, so as to automatically perform at least two types of operational functions in the orthopedic surgery, such as an automatic drilling function and an automatic screw placement function. The surgical operational functions may also include osteotomy, joint replacement, bone traction, debridement, insertion and removal of Kirschner wires, or other surgical operational functions. For example, in order to implement functions such as drilling and screw placement, for the first robotic arm 301 and the second robotic arm 302, firstly real-time monitoring and guidance of the operations of the first robotic arm 301 and the second robotic arm 302 are performed by the positioning-and-navigation system based on the trajectories for drilling and screw placement obtained from preoperative planning, thereby ensuring the precise control of the first robotic arm 301 and the second robotic arm 302 to complete drilling- and-screw-placement operations. In particular, in a non-limiting embodiment of the present disclosure, it ensures the precise control of screw placement by the first robotic arm 301 and the drilling and screw placement that are sequentially performed by the second robotic arm 302.

The third robotic arm 303 may be configured to fix the imaging device 201 and is communicatively connected to a data-analysis-and-processing module 101 and a robotic arm control module 103 as shown in Fig. 2 for positioning and orienting the imaging device 201, so as to enable the imaging device 201 to maintain a working distance and an orientation overlooking the surgical site throughout the orthopedic surgical procedure.

The control device 100 for controlling the robotic arm device 300 is communicatively coupled with the robotic arm device 300 and the positioning-and-navigation device 200, respectively; and the control device 100 mainly includes a data-analysis-and-processing module 101, a surgical planning module 102 and a robotic arm control module 103, which will be further described below. The control device 100 may be implemented as a computer device with a computer-readable storage medium as shown in Fig. 1, and the positioning-and-navigation device 200 includes an electromagnetic navigation device 210 and an optional infrared coherent imaging navigation device 220, which are not shown in Fig. 1 but will be further described below.

The electromagnetic navigation device 210 is configured to position at least two of the three end effectors 400 in a non-limiting embodiment of the present disclosure within the surgical site, and the electromagnetic navigation device 210 mainly includes an unshown tracking device for electromagnetic tracking, a reference frame and a medical imaging equipment for electromagnetic navigation that are not shown in Fig. 1. The medical imaging equipment is used to perform an intraoperative scan of the surgical site and the reference frame to acquire coordinate positions of the surgical site and a marker based on a coordinate system of the medical imaging equipment, and the medical imaging equipment may in particular be implemented as a CT device.

The tracking device, not shown in the figures, includes a magnetic field generator for generating a magnetic field and at least one electromagnetic tracking element. The at least one electromagnetic tracking element includes a first electromagnetic tracking element and a second electromagnetic tracking element. The first electromagnetic tracking element may be embedded in an end effector 400 of the orthopedic surgical robot 10 or embedded in an implant adapted to the end effector. Alternatively, the first electromagnetic tracking element may be mounted on an end effector 400 of the orthopedic surgical robot 10 or mounted on an implant adapted to the end effector without affecting the surgical effect, and the second electromagnetic tracking element is arranged on the reference frame of the positioning-and-navigation device. The first electromagnetic tracking element continuously tracks the position and orientation of the end effectors 400 or the implants adapted to the end effectors throughout the surgical procedure. The electromagnetic navigation device 210 further includes a magnetic field generator for generating a magnetic field, which may be arranged at these positions so that its magnetic field covers the positions of the at least one electromagnetic tracking element, provided that it does not interfere with the movement of the orthopedic surgical robot 10 or typical preoperative or intraoperative workflows in an operating room. Advantageously, the magnetic field generator may be arranged on the patient support device 500, and preferably integrated into the patient support device 500.

The reference frame may be detachably fixed to rigid tissue closest to the surgical site, and the rigid tissue may be a spinous process or other bony tissue of a patient to undergo orthopedic surgery. The second electromagnetic tracking element and the marker that is capable of displaying a coordinate position in an image of the medical imaging equipment are detachably fixed to the reference frame. The medical imaging equipment may be configured to perform the intraoperative scan of the surgical site and the reference frame to acquire the coordinate positions of the surgical site and the marker based on the coordinate system of the medical imaging equipment.

The control device 100 may further control the magnetic field generator to generate a magnetic field, in particular a three-dimensional magnetic field, thereby acquiring the position of the second electromagnetic tracking element arranged on the reference frame. The magnetic field generator may generate a magnetic field above the target area to be operated on, covering both the second electromagnetic tracking element on the reference frame and the first electromagnetic tracking element. The medical imaging equipment may perform an intraoperative scan, in particular a three-dimensional scan, of the target area and the reference frame to acquire the coordinate positions, in particular three-dimensional coordinate positions, of the marker on the reference frame in the target area based on the coordinate system of the imaging device.

The working principle of the electromagnetic positioning-and-navigation device according to the present disclosure is preferably as follows: first, an intraoperative three-dimensional CT scan of the surgical site and the reference frame is performed by a CT scanner (not shown) to acquire coordinate positions of the target area and the marker on the reference frame based on the CT coordinate system; next, the control device 100 controls the magnetic field generator to generate a three-dimensional magnetic field to acquire the position of the second electromagnetic tracking element on the reference frame; because a relative position between the marker on the reference frame and the second electromagnetic tracking element on the reference frame is known, CT coordinates and electromagnetic field coordinates can be registered based on this relative position relationship; in addition, the position relationships between the first electromagnetic tracking elements at the first, second and third robotic arms, the end effector 400, and the free end of the imaging device 201 are known, coordinate registration between the electromagnetic field coordinate and the coordinates of the first end effector 410, the second end effector 420, and the imaging device 201 can be performed through these positional relationships; finally, registration of the CT coordinates, the electromagnetic field coordinates, the coordinates of the first end effector 410 and the second end effector 420, and the coordinates of the imaging device 201 is achieved.

In order to achieve more precise positioning and anatomical visualization of the surgical robot 10 within the surgical site, the positioning-and-navigation device 200 further additionally includes an imaging device 201 and a monitoring device in a non-limiting embodiment of the present disclosure.

The imaging device 201 may include an infrared coherent imaging navigation device 220 and a remote microscopic device 2011. The remote microscopic device 2011 is capable of providing surgeons and nurses with visualization of the surgical site to monitor the safe and smooth progress of the surgical procedure. The infrared coherent imaging navigation device 220 is capable of providing more accurate positioning and navigation for the surgical robot 10, because the infrared coherent imaging navigation device 220, in conjunction with the data-analysis-and-processing module 101, can be used to locate the surgical site, the first robotic arm 301 and the second robotic arm 302, and enables the first robotic arm 301 and the second robotic arm 302 to determine their positions relative to the patient.

The remote microscopic device 2011 is capable of implementing an imaging function and tracking the surgical site by an identifier for the surgical site, so as to allow surgical participants, in particular surgeons, to clearly visualize the surgical site. The remote microscopic device 2011 is preferably arranged at a position more than 1 meter above the surgical table, which ensures image acquisition of objects within the surgical site, thereby facilitating intraoperative surgical monitoring and learning by medical staff, avoiding the conflict with other intraoperative instruments and devices, and eliminating the need for sterile draping. The tracking function is implemented by the data-analysis-and-processing module 101 shown in Fig. 2, which tracks an identifier (in particular, a unique identifier) that is used for identifying the surgical site through algorithms. The distance, position and orientation of the identifier are used as the input to the robotic arm control module 103 in Fig. 2 to control the position and orientation of the robotic arm. In addition, the imaging device 201 may also provide advanced stereoscopic three-dimensional visualization of the surgical site, enabling the natural depth perception required for complex orthopedic surgeries.

In a non-limiting embodiment of the present disclosure, the monitoring device is implemented as a plurality of monitors, preferably a first monitor 203 and a second monitor (not shown). The first monitor 203 is configured to display data from the infrared coherent imaging navigation device 220 and the electromagnetic navigation device 210, so as to facilitate doctors and nurses to monitor the surgical progress respectively during the surgical operation and to timely prevent unexpected motion trajectories of the end effector, particularly a surgical tool, during the surgical procedure. In a non-limiting embodiment of the present disclosure, the first monitor 203 is implemented as a small monitor, preferably the number is designed to be two, so that doctors and nurses at different positions can better view the necessary information relevant to their respective surgical work. Moreover, the second monitor, not shown in Fig. 1, is configured to display data from the remote microscopic device 2011 and is implemented as a large medical-grade surgical monitor, such as a 50-inch or larger monitor, which can provide an opportunity for everyone in the operating room to view the surgical site, especially for doctors, orthopedic surgery students, and other personnel to more intuitively view the magnified surgical site. For this reason, the second monitor may advantageously be movably arranged beside the cart device 600. The above-mentioned "data" forms include visual images, medical images captured preoperatively and intraoperatively, coordinates, angles, orientations, or other monitored data.

The surgical robot 10 is generally further equipped with a tool set of the orthopedic surgical robot 10 for orthopedic surgery, which includes, for example, an end effector 400, an adapter device, an implant, and an implant-inserter that is used for placing the implant into the patient's body. For example, in the case of spinal surgery, the implant-inserter is preferably configured as a screw driver. Alternatively, the implant-inserter may also be a device that assists in placing the interbody fusion cage into the body.

The adapter device includes a first adapter 4011 and a second adapter 4012 connectable to the ends of the first robotic arm 301 and the second robotic arm 302, respectively. The first robotic arm 301 can be coupled with a first implant-inserter for a first implant and/or a second implant-inserter for a second implant through the first adapter 4011, and the second robotic arm 302 can be coupled with a drilling tool through the second adapter 4012. The first implant-inserter may be the same as the second implant-inserter, or may be an implant-inserter different in type and model. Generally, adapters corresponding to different implant-inserters are different, but a multi-purpose adapter adapted to different implant-inserters may also be designed. In a non-limiting embodiment of the present disclosure, the first implant-inserter and the second implant-inserter are the same, and are further each configured as a screw driver as described above.

In a non-limiting embodiment of the present disclosure, first, the second robotic arm 302 is coupled with an osteotome serving as a drilling tool through the second adapter 4012, so as to perform drilling prior to screw placement. Then, the second robotic arm 302 is coupled with a screw driver acting as one of the end effectors 400 through the first adapter 4011, so as to reduce the degree of physician involvement in the orthopedic surgery or, in other words, to increase the degree of automation of the orthopedic surgery.

The patient support device 500 is generally configured in the form of a surgical table, which is used to support the patient to be operated on and to mount the magnetic field generator for electromagnetic positioning and navigation.

The surgical robot 10 for orthopedic surgery generally further includes a cart device 600, which is configured to place the control device 100 and components required for the control device 100, and to connect and fix the robotic arm device 300, and the monitoring device, and the like.

Fig. 2 shows a block diagram of the working principle of a surgical robot 10 for orthopedic surgery according to a non-limiting embodiment of the present disclosure. The surgical robot 10 of the present disclosure for orthopedic surgery, particularly spinal screw placement surgery, is equipped with a positioning-and-navigation device 200, schematically indicated by a dashed circle, which adopts a dual navigation system, namely an electromagnetic navigation system and an optional infrared coherent imaging navigation system, so as to select an optimal navigation mode adapted to specific surgical conditions, such as minimally invasive surgery or open surgery, for example, using only the electromagnetic navigation mode or a combined mode of electromagnetic navigation and infrared coherent imaging navigation. Among them, with regard to electromagnetic navigation, the electromagnetic navigation can be applied to both minimally invasive and open surgeries. In contrast, with regard to infrared coherent imaging navigation, although the infrared coherent imaging navigation is more suitable for open surgery due to its imaging depth limitation, its high-resolution imaging capability enables ultra-high-precision navigation.

As shown on the left side of Fig. 2, an electromagnetic navigation device 210 is used to implement electromagnetic navigation. The electromagnetic navigation device 210 is equipped with an electromagnetic navigation reference frame and supporting surgical tools. Positioning and tracking are achieved by acquiring position data of the surgical site and position data of the surgical tool through electromagnetic detection elements, and navigation is achieved by registering with CT imaging coordinates and robotic arm system coordinates. In order to realize the optional infrared coherent imaging navigation, an infrared coherent imaging navigation device 220 is used, and the coherent distance of the infrared coherent imaging navigation device 220 reaches more than 1 meter through optical design, thereby enabling long-distance real-time coherent imaging of the surgical site and surgical tools or instruments. Tracking and navigation can be achieved after the infrared coherent imaging navigation device 220 is coordinate-registered with the robotic arm. The data-analysis-and-processing module of the control device 100 receives relevant data from the electromagnetic navigation device and the infrared coherent imaging navigation device, thereby enabling real-time navigation registration and, in particular, also achieving suppression of drift caused by patient respiration.

If a combined mode of electromagnetic navigation and infrared coherent imaging navigation is adopted, medical imaging, particularly CT imaging, is not required intraoperatively, which can reduce the X-ray radiation exposure to doctors and patients, while also enabling precise robotic positioning and anatomical visualization within the surgical site, thereby assisting surgeons and/or the surgical robot 10 in performing the surgery better during the surgical procedure.

The data-analysis-and-processing module 101 of the control device 100 is mainly configured to analyze and process input data from the electromagnetic navigation device 210, the imaging device 201, and the medical imaging equipment, so as to assist the orthopedic surgery robot 10 in performing automatic orthopedic surgery on a patient, and to assist surgeons in surgical planning, and teaching-research by extracting various forms of functional information. The input data in the data-analysis-and-processing module includes, but is not limited to, visual image data from the imaging device 201 and surgical site imaging data. The surgical site imaging data includes data from the electromagnetic navigation device 210 and medical image data from preoperative/intraoperative medical imaging 230 of the patient, where the preoperative/intraoperative medical imaging of the patient is especially selected as preoperative/intraoperative CT imaging.

The surgical planning module 102 of the control device 100 can receive target area data from the data-analysis-and-processing module 101 and can be used for surgical planning, so as to ensure rapid and seamless surgical operations during the orthopedic surgery. During the planning phase of the surgical planning module, a user can select and save, through an operable interface, cooperating objects such as screws or other orthopedic surgical instruments planned for the orthopedic surgery, particularly entry points and trajectories of implants. Herein, the screws may be, for example, spinal screws in spinal surgery in non-limiting embodiments of the present disclosure. The saved positions and orientations of the cooperating objects of the screws or other orthopedic surgical instruments, particularly the trajectories of implants, are shared with the robotic arm control module 103 in the form of coordinate transformation for determining the motion trajectory of the corresponding robotic arm.

The robotic arm control module 103 of the control device 100 is configured to receive an analysis result from the data-analysis-and-processing module 101, such as a target pose of the robotic arm, and control the corresponding robotic arm to perform actions accordingly. Specifically, the robotic arm control module 103 controls the movement of the at least two robotic arms and the at least two end effectors based on the target pose of the robotic arm from the surgical planning module 102 and the analyzed and processed results from the data-analysis-and-processing module 101, such as the target pose of the robotic arm. In addition, the robotic arm control module 103 is further configured to define movement limit of the robotic arm and position limit in the form of "forbidden zones" through angles and Cartesian coordinates, so as to ensure the safety and health of the patient and surgical medical staff working near the robotic arm.

Figs. 3a-3b are non-limiting embodiments of a first implant and a second implant according to the present disclosure, respectively. The implants according to the present disclosure include a first implant and a second implant, where the first implant is configured as a left-handed pedicle screw as shown in FIG. 3a, and the second implant is configured as a right-handed pedicle screw as shown in FIG. 3b, so as to fix the spine, in particular to restore the alignment of the spine and maintain the stability of the spine.

In a scenario requiring the placement of left-handed or right-handed screws, for example, in a case where the bone is very hard and using only right-handed screws may cause secondary injuries such as fractures, after drilling a hole through the second robotic arm 302, the second adapter 4012 may be replaced with the first adapter 4011 and connected to a screw driver, and then the left-handed pedicle screw 4031 or the right-handed pedicle screw 4032 may be fixed, so as to achieve simultaneous insertion of left-handed and right-handed pedicle screws in cooperation with the first robotic arm 301. The left and right torque balance control is performed by the robotic arm control module 103, thereby achieving torque cancellation and thus accurately placing the pedicle screws.

While examples of the present disclosure are provided in the foregoing description, modifications and variations may be made to these examples by those skilled in the art without departing from the scope and spirit of the present disclosure. For example, it should be understood that features of the embodiments herein may be applied to other embodiments described herein. Accordingly, the description is intended to be illustrative rather than restrictive. The present disclosure is defined by the appended claims, and all changes to the present disclosure that come within the meaning and range of equivalency of the claims are to be embraced within their scope.

## Claims

1. A surgical robot for orthopedic surgery, comprising: a robotic arm device, a positioning-and-navigation device, and a control device that is communicatively coupled with the robotic arm device and the positioning-and-navigation device, respectively, wherein the robotic arm device is provided with at least two robotic arms, an end effector is mounted at an end of each of the at least two robotic arms proximate to a surgical site, the positioning-and-navigation device comprises an electromagnetic navigation device, and the electromagnetic navigation device comprises:
a tracking device for electromagnetic tracking, comprising: a magnetic field generator configured to cover at least two end effectors, and at least two first electromagnetic tracking elements for respectively tracking orientations of the at least two end effectors during a surgery; and
a reference frame, wherein a second electromagnetic tracking element and a marker that is capable of displaying a coordinate position are mounted on the reference frame,
wherein the positioning-and-navigation device further comprises an imaging device for positioning and navigating the at least two end effectors, and the imaging device is provided with an infrared coherent imaging navigation device for positioning the surgical site and the at least two end effectors.

2. The surgical robot according to claim 1, wherein the imaging device is arranged at an end of one of the at least two robotic arms that is proximate to the surgical site, and the imaging device is communicatively connected to the control device, so as to maintain the imaging device in a working position and orientation throughout a surgical procedure.

3. The surgical robot according to claim 1 or 2, wherein the imaging device further comprises a remote microscopic device for imaging and tracking the surgical site, and the remote microscopic device is arranged at a position more than 1 meter above a patient support device.

4. The surgical robot according to claim 3, wherein the control device is configured to perform the tracking by an identifier for the surgical site.

5. The surgical robot according to claim 4, wherein the control device is configured to control orientations of the at least two robotic arms based on a distance of the identifier and an orientation of the identifier.

6. The surgical robot according to claim 3, wherein the positioning-and-navigation device further comprises a monitoring device with a plurality of monitors, one of the plurality of monitors is a first monitor for displaying data from the infrared coherent imaging navigation device and the electromagnetic navigation device, and another one of the plurality of monitors is a second monitor for displaying data from the remote microscopic device.

7. The surgical robot according to claim 3, wherein the remote microscopic device is configured for stereoscopic visualization of the surgical site to perceive the surgical site in depth preoperatively.

8. The surgical robot according to claim 3, wherein the at least two robotic arms comprise: a first robotic arm for mounting a first end effector, a second robotic arm for mounting a second end effector, and a third robotic arm for mounting the imaging device.

9. The surgical robot according to claim 8, wherein the remote microscopic device and the infrared coherent imaging navigation device are arranged on the third robotic arm, so as to enable a visual field of the remote microscopic device and a visual field of the infrared coherent imaging navigation device to coincide.

10. The surgical robot according to claim 8, wherein the first end effector is configured as a first implant-inserter for implanting a first implant into a patient, and the second end effector is selectively configured as a second implant-inserter for implanting a second implant into the patient or as a drilling tool.

11. The surgical robot according to claim 10, wherein the second end effector is configured as a second implant-inserter, the first implant-inserter cooperates with the second implant-inserter during the surgical procedure, so as to enable torques exerted by the first implant-inserter and the second implant-inserter on the surgical site to be balanced.

12. The surgical robot according to claim 11, wherein the first implant is configured as one of a left-handed pedicle screw and a right-handed pedicle screw, and the second implant is configured as the other one of the left-handed pedicle screw and the right-handed pedicle screw.

13. The surgical robot according to claim 1 or 2, wherein the control device comprises:
a surgical planning module configured for planning a surgery;
a data-analysis-and-processing module configured for analyzing and processing data to control the robotic arm device;
a robotic arm control module configured for controlling movement of the robotic arm device, wherein the surgical planning module is configured to select and save an entry point and a trajectory for a planned orthopedic surgical operation during a planning phase.

14. The surgical robot according to claim 13, wherein the data-analysis-and-processing module is configured to share, in a form of coordinate transformation, a position of the entry point and an orientation of the trajectory for the orthopedic surgical operation that are selected and saved with the robotic arm control module, so as to determine a motion trajectory of a corresponding robotic arm.

15. The surgical robot according to claim 13, wherein the robotic arm control module is further configured to define motion orientations of the at least two robotic arms by angles and Cartesian coordinates.
